# EUROPEAN PATENT SPECIFICATION

(11) **EP 1 638 598 B1**
(45) Date of publication and mention of the grant of the patent: **06.12.2006**
(21) Application number: 04777430.2
(22) Date of filing: 02.07.2004
(51) Int. Cl.: A61K 39/102, C12N 1/20

(54) **ACAPSULAR i P. MULTOCIDA HYAE /i DELETION MUTANTS**
AKAPSULÄRE I P. MULTOCIDA HYAE /I DELETIONSMUTANTEN
MUTANTS ACAPSULAIRES DE DELETION D'UN GENE HYAE DE I P /I . MULTOCIDA

(30) Priority: 02.07.2003 US 608931 P
(43) Date of publication of application: 29.03.2006
(73) Proprietor: BIOTECHNOLOGY RESEARCH AND DEVELOPMENT CORPORATION, Peoria, IL 61604 (US); THE UNITED STATES OF AMERICA as represented by THE SECRETARY, UNITED STATES DEPARTMENT OF AGRICULTURE, Washington, DC 20250-0302 (US)
(72) Inventor: BRIGGS, Robert, E., Boone, IA 50036 (US); TATUM, Fred, M., Ames, IA 50010 (US)
(74) Representative: Tombling, Adrian George
(86) International application number: PCT/US2004/021274
(87) International publication number: WO 2005/003330

(56) References cited:
- EP-A- 1 350 796
- WO-A-03/086277
- CHUNG J Y ET AL: "THE CAPSULE BIOSYNTHETIC LOCUS OF PASTEURELLA MULTOCIDA A:1" FEMS MICROBIOLOGY LETTERS, AMSTERDAM, NL, vol. 166, 15 September 1998 (1998-09-15), pages 289-296, XP002921439 ISSN: 0378-1097
- ADLER B ET AL: "Candidate vaccine antigens and genes in Pasteurella multocida" JOURNAL OF BIOTECHNOLOGY, ELSEVIER SCIENCE PUBLISHERS, AMSTERDAM, NL, vol. 73, no. 2-3, 20 August 1999 (1999-08-20), pages 83-90, XP004180171 ISSN: 0168-1656
- CHUNG JING YENG ET AL: "Role of capsule in the pathogenesis of fowl cholera caused by Pasteurella multocida serogroup A" INFECTION AND IMMUNITY, vol. 69, no. 4, April 2001 (2001-04), pages 2487-2492, XP002308293 ISSN: 0019-9567

## Description

### FIELD OF THE INVENTION

The invention relates to acapsular mutants of *Pasteurella multocida* and vaccines comprising the mutants.

### BACKGROUND OF THE INVENTION

*Pasteurella multocida* (*P. multocida*) is associated with a variety of diseases, including calf and yearling meningoencephalitis, lamb lymphadenitis, horse and donkey septicemia, bovine septicemic pasteurellosis (hemorrhagic septicemia, barbone), swine pasteurellosis, porcine septicemic pasteurellosis, pneumonia, and fowl cholera.

There is a need in the art for effective vaccines that can be used to provide protective immunity against diseases caused by *P. multocida.*

### SUMMARY OF THE INVENTION

One embodiment of the invention is an isolated *Pasteurella multocida* (*P. multocida*) bacterium of serogroup A which comprises a deletion of all or a part of a *hyaE* gene. The deletion attenuates the bacterium.

Another embodiment of the invention is a vaccine for inducing protective immunity against wild-type *P. multocida.* The vaccine comprises *P. multocida* bacterium of serogroup A which comprises a deletion of all or a part of a *hyaE* gene and a pharmaceutically acceptable vehicle. The deletion mutation attenuates the bacterium.

Yet another embodiment of the invention are feeds suitable for mammals (including livestock, ungulates, and companion animals or birds (preferably poultry) comprising a *P*. *multocida* bacterium of serogroup A which comprises a deletion of all or a part of a hyaE gene. The mutation attenuates the bacterium.

Even another embodiment of the invention is the use of a P. multocida bacterium of serogrup A which comprises a deletion of all or a part of a hyaE gene, which attenuates the bacterium, in the manufacture of a medicament for inducing protective immunity against wild-type P. multocida. The bacterium is for administration to an animal subject such as a mammal (including livestock, ungulates and companion animals) or a bird (including poultry). The bacterium thereby confers to the animal subject protective immunity against wild-type *P. multocida.*

The invention thus provides tools and methods for inducing protective immunity against diseases caused by *P. multocida.*

### BRIEF DESCRIPTION OF THE FIGURE

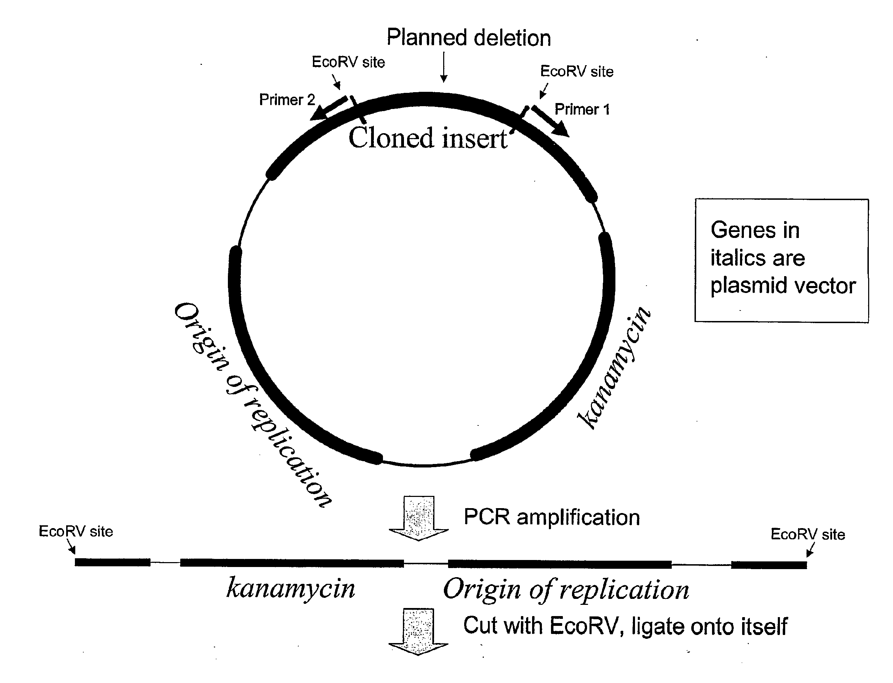

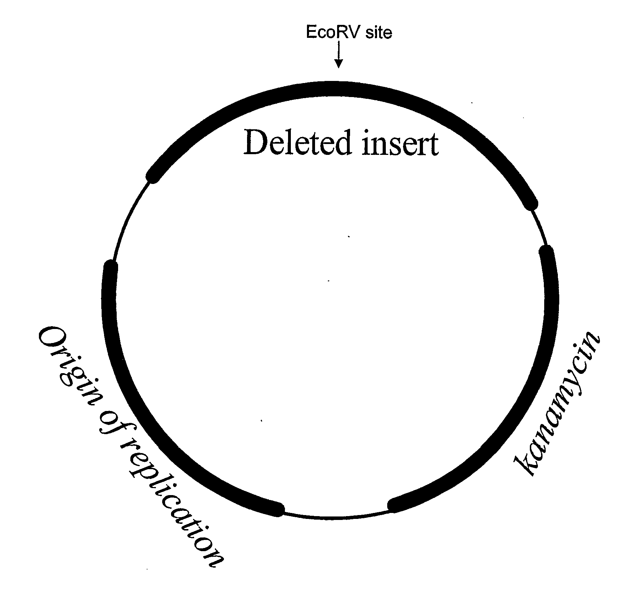

FIG. 1. Illustration of the construction of *hyaE* deletion mutants. FIG. 1A, schematic showing planned deletion. FIG. 1 B, plasmid containing deleted *hyaE* insert.

### DETAILED DESCRIPTION OF THE INVENTION

Acapsular *hyaE* deletion mutants of *P. multocida* serogroup A can be administered to mammals (including livestock, ungulates, and companion animals) and birds (including poultry) to provide protective immunity against wild-type *P. multocida, e.g.,* to prevent or reduce the severity of diseases such as hemorrhagic septicemia or pneumonia in livestock, ungulates, and companion animals and to prevent or reduce the severity of fowl cholera in birds, especially poultry, respectively. The terms "acapsular mutant(s)," "acapsular bacterium(a)," and "mutant bacterium(a)" are used interchangeably in this description.

### Acapsular hyaE deletion mutants of P. multocida serogroup A

Acapsular *hyaE* deletion mutants of serogroup A *P. multocida* (*e.g.,* serotypes A:1, A:3, A:4) can be generated by mutagenizing the *hyaE* gene in the capsule biosynthetic locus. The genes of the capsule biosynthetic locus in serogroup A are well known and have been completely sequenced. Chung *et al., FEMS Microbiol. Lett. 166(2),* 289-96, 1998. The serotype A:1 locus contains four open-reading frames (ORFs) in region 1 (*hexA, hexB, hexC,* and *hexD*)*,* five ORFs in region 2 (*hyaA, hyaB, hyaC, hyaD,* and *hyaE*)*,* and two ORFs in region 3 (*phyA* and *phyB*)*.*

In certain embodiments, the mutant bacteria do not comprise any antibiotic resistance genes or foreign DNA, which improves their environmental and ecological attractiveness. A method of generating deletion mutants is described in Example 1, but any other methods known in the art can be used to generate deletion mutations. Simple tests for confirming that mutants have an acapsular phenotype also are disclosed in Example 1.

*P. multocida* bacteria with any deletion of all or a part of the *hyaE* gene is within the scope of the invention so long as the deletion attenuates the bacterium. In one embodiment, the deletion mutation results in the deletion of amino acids 239-359 of the hyaE protein (i.e., none of amino acids 239-359 are present in the encoded hyaE protein). In other embodiments, the mutated *hyaE* open reading frame comprises SEQ ID NO:7 or SEQ ID NO:8.

A mutant bacterium is attenuated if, after exposure to the mutant bacterium, an increase in the dosage of wild-type *P. multocida* is required to kill half the susceptible target species (*i.e.,* the LD₅₀ increases) and/or there is a reduction in pathologic lesions (*e.g.,* pneumonia) after exposure of the target species to the mutant bacterium compared with exposure to a wild-type bacterium, and/or there is a reduction in commensal colonization of mucosal surfaces where *P. multocida* reside after exposure of the target species to the mutant bacterium when compared with exposure to a wild-type bacterium.

Attenuation can be assessed by various means as is known in the art. For example, for septiceaemic disease (such as fowl cholera), susceptible species can be exposed to wild-type organisms by intranasal, intravenous, intramuscular, or intraperitoneal routes, and the dosage required to cause disease between wild-type and mutant organisms can be compared. For pneumonic disease, one can expose susceptible animals to wild-type organisms by intranasal, intratracheal, or intrapulmonic routes and compare the extent and severity of clinical symptoms and pathologic lesions between mutant- and wild-type-exposed animals. For mucosal colonization, animals can be exposed to wild-type organisms by oral, intranasal, or intratonsillar routes and the numbers of organisms recovered from nasal mucus or tonsillar wash specimens at intervals after exposure can be compared.

### Vaccine preparations

Vaccines comprising mutant bacteria can be given alone or as a component of a polyvalent vaccine, *i.e*., in combination with other vaccines. Mutant bacteria in a vaccine formulation can be live or killed; either live or killed bacteria can be lyophilized and, optionally, reconstituted as is known in the art. Vaccines can conveniently be provided in kits, which also can comprise appropriate labeling and instructions for administering a vaccine to an animal subject (*e.g.,* livestock, an ungulate, a companion animal) or a bird (*e.g.,* poultry).

Vaccines comprising acapsular mutants also can comprise pharmaceutically and veterinarily acceptable carriers. Such carriers are well known to those in the art and include, but are not limited to, large, slowly metabolized macromolecules, such as proteins, polysaccharides, polylactic acids, polyglycolic acids, polymeric amino acids, amino acid copolymers, and inactive virus particles. Pharmaceutically and veterinarily acceptable salts can also be used in the vaccine, for example, mineral salts such as hydrochlorides, hydrobromides, phosphates, or sulfates, as well as the salts of organic acids such as acetates, proprionates, malonates, or benzoates. Vaccines also can contain liquids, such as water, saline, glycerol, and ethanol, as well as substances such as wetting agents, emulsifying agents, or pH buffering agents. Liposomes also can be used as carriers for mutant bacteria. See U.S. Patent 5,422,120, WO 95/13796, WO 91/14445, or EP 524,968 B1.

If desired, an adjuvant can be added to a vaccine. Useful adjuvants include, without limitation, surfactants (*e.g.,* hexadecylamine, octadecylanine, lysolecithin, dimethyldioctadecylammonium bromide, N,N-dioctadecyl-n'-N-bis(2-hydroxyethyl-propane di-amine), methoxyhexadecylglycerol, and pluronic polyols); polyanions (*e.g.,* pyran, dextran sulfate, poly IC, polyacrylicacid, carbopol), peptides (*e.g.,* muramyl dipeptide, dimethylglycine, tuftsin), oil emulsions, alum, and mixtures thereof

### Treatment of mammals, particularly livestock, ungulates, and companion animals

"Mammals" include monotremes *(e.g.,* platypus), marsupials *(e.g.,* kangaroo), and placentals, which include livestock (domestic animals raised for food, milk, or fiber such as hogs, sheep, cattle, and horses) and companion animals (*e.g*., dogs, cats). "Ungulates" include, but are not limited to, cattle (bovine animals), water buffalo, bison, sheep, swine, deer, elephants, and yaks. Each of these includes both adult and developing forms (*e.g*., calves, piglets, lambs, etc.). Bacteria of the invention can be administered either to adults or developing mammals, preferably livestock, ungulates, or companion animals.

A convenient method of delivering a bacterium of the invention to mammals (such as livestock, ungulates, or companion animals) is by oral administration (*e.g*., in the feed or drinking water or in bait). It is particularly convenient to top-dress or mix feed with the bacteria. Typically, large animals (*e.g*., livestock/ungulates such as cattle) are dosed with about 10⁶, 5 x 10⁶, 10⁷, 5 x 10⁷, 10⁸, 5 x 10⁸, 10⁹, 5 x 10⁹, or 10¹⁰ cfu; about 10⁸, 5 x 10⁸, 10⁹, 5 x 10⁹ cfu if feed is top-dressed. Doses of about 10⁶ to about 10⁸, about 2 x 10⁶ to about 3 x 10⁸, about 2.4 x 10⁶ to about 2.6 x 10⁸, about 10⁴ to about 10⁶ cfu or of about 10⁴ to about 10⁹ cfu can be given. Doses can be adjusted for smaller livestock/ungulates such as sheep (*e.g.,* about 10⁴, 5 x 10⁴, 10⁵, 5 x 10⁵, 10⁶, 5 x 10⁶, 10⁷, 5 x 10⁷, 10⁸, 5 x 10⁸ cfu). Analogous dosing regimens can be readily deduced for companion animals.

Although the oral route is preferred for ease of delivery, other routes for vaccination can also be used. These include without limitation, subcutaneous, intramuscular, intravenous, intradermal, intranasal, intrabronchial, etc. Bacteria of the invention can be implanted in the ear. Bacteria also can be administered by airspray, by eye inoculation, or by scarification.

### Treatment of birds

"Birds" include wild (*e.g.,* game fowl) and domesticated (*e.g.,* poultry or pet) birds and includes both adult and developing forms (*e.g*., hatchlings, chicks, poults, etc.). "Poultry" or "poultry birds" include all birds kept, harvested, or domesticated for meat or eggs, including chicken, turkey, ostrich, game hen, squab, guinea fowl, pheasant, quail, duck, goose, and emu.

Bacteria of the invention can be administered to a bird by any known or standard technique, including mucosal or intramuscular injection. In a hatchery, bacteria can be administered using techniques such as *in ovo* vaccination, spray vaccination, or subcutaneous vaccination. On the farm, bacteria can be administered using techniques such as scarification, spray vaccination, eye drop vaccination, in-water vaccination, infeed vaccination, wing web vaccination, subcutaneous vaccination, and intramuscular vaccination.

Effective doses depend on the size of the bird. Doses range and can vary, for example, from about 10², 5 x 10², 10³, 5 x 10³, 10⁴, 5 x 10⁴, 10⁵, 5 x 10⁵, 10⁶, 5 x 10⁶, 10⁷, 5 x 10⁷, 10⁸, 5 x 10⁸, 10⁹, to 5 x 10⁹ cfu.

The above disclosure generally describes the present invention. A more complete understanding can be obtained by reference to the following specific examples, which are provided for purposes of illustration only and are not intended to limit the scope of the invention.

### EXAMPLE 1

### Generation of acapsular hyaE deletion P. multocida mutants

*P. multocida* mutants of strains 1059 (avian, serotype A:3) and 1062 (bovine, serotype A:3) were generated by deleting the coding region of their *hyaE* genes, which blocked synthesis of the capsular building block *N*-acetyl-D-glucosamine. The coding regions of the 1059 and 1062 *hyaE* genes (SEQ ID NOS:5 and 6, respectively) were obtained by PCR amplification, using the forward primer 5'-ATGAAAAAGGTTAATATCATTGG-3' (SEQ ID NO:1) and the reverse primer 5'-TTAACCTTGCTTGAATCGTTTACC-3' (SEQ ID NO:2). All primers were synthesized with an oligonucleotide synthesizer (Applied Biosystems Inc.) by Integrated DNA Technologies, Inc., Coralville, IA. The PCR reactions were carried out using the GeneAmp LX PCR Kit (PE Applied Biosystems, Foster City, CA) in a Perkin Elmer GeneAmp 9600 thermocycler. Reaction conditions were 30 cycles, with 30 seconds at 95°C, 45 seconds at 48°C, and 60 seconds at 72°C per cycle.

The two PCR-generated *hyaE* fragments initiated at their start Met codons and ended at their stop codons. The PCR fragments were ligated into pCR2.1 (Invitrogen Inc., LaJolla, CA) and electroporated into the *E. coli* strain DH11S (Life Technologies, Rockville, MD), which generated the plasmids pCR2.1*hyaE*1059 and pCR2.1*hyaE*1062. The two plasmid constructs were isolated by the alkaline SDS method, then purified by CsCl centrifugation using standard methods. Both strands of both *hyaE* genes were sequenced using the Dye Terminator Chemistry kit from PE Applied Biosystems. Samples were run on an ABI Prism 377 DNA Sequencer by the Nucleic Acids Facility, Iowa State University, Ames, IA.

The structural *hyaE* genes of each strain were 1869 bp in length. Due to sequence variation, primarily found in the 3' end of the coding region, unique *hyaE* replacement plasmids were constructed for each strain in order to engineer the two mutant strains of *P*. *multocida.* Precise deletions within each *hyaE* gene contained on plasmids pCR2.1*hyaE*1059 and pCR2.1*hyaE*1062 were achieved by PCR using the deletion primers 5'-AAAGATATCTTGGTTTACTTCAATAATTTC-3' (SEQ ID NO:3) and 5'-AAAGATATCACTGCATCTGTTCAATCAACGAGC-3' (SEQ ID NO:4). The deletion primers anneal to the cloned gene approximately 300 base-pairs apart but extend outwards, towards the plasmid vector, rather than inwards. The PCR reaction amplifies the entire cloning vector and two ends of the gene insert but not the approximately 300 base pair "deletion." The amplified product is linear, with the two *hyaE* gene fragments on the ends and plasmid vector in the middle. The primers contain EcoRV sites (GATATC) on their 5' termini. The ends of the linear amplified product, therefore, contain EcoRV sites. See Figures 1A and 1B.

The resulting PCR fragments were subjected to *Eco*RV restriction digestion to remove specific sequences from their ends. Each fragment was then ligated to generate inframe deletions excising nucleotides 715 through 1082. An eight basepair *Sma*I linker (5'-CCCCGGGG-3') was inserted into the *Eco*RV deletion site of *P. multocida* 1062 to ensure that the mutation would result in an acapsular phenotype. No such DNA was inserted into the deletion site of the *P. multocida* 1059 *hyaE* insert.

The nucleotide sequences of the 1059 and 1062 mutated *hyaE* fragments are shown in SEQ ID NOS:7 and 8, respectively. In both 1059 and 1062, amino acid 239 through amino acid 359 of the hyaE protein were deleted. In addition, in both strains the amino acid at former position 360 was changed from leucine to isoleucine. Strain 1062 received an additional 8 amino acids in the deletion site just upstream from the former position 360 (Pro Arg Gly Pro Gly Ala Pro Gly; SEQ ID NO:9).

The mutated *hyaE* fragments were subcloned into *Eco*RI sites within the multiple cloning site of plasmid pBCSK (Strategene Inc.), followed by insertion of the Tn903 kanamycin resistance element into the adjacent *Bam*HI sites to produce pBCSKΔ*hyaE*kan^{r}1059 and pBCSKΔ*hyaE*kan^{r}1062, respectively. Construction of the replacement plasmids were completed by ligating *Bss*H2 generated Δ*hyaE*kan^{r} fragments of 1059 and 1062 with the 1.2 kb temperature-sensitive origin of replication of pBB 192 excised from the *Bss*H2 site of pBCSK (Stratagene, Inc.). See U.S. Patent 5,840,556. Because ColE1 origin is inactive in *P. multocida,* only the ligation products generating plasmids p192oriΔ*hyaE*kan^{r}1059 or p192oriΔ*hyaE*kan^{r}1062 were capable of replicating within the hosts.

Replacement plasmids were introduced into the appropriate *P. multocida* strains by electroporation. Cells were grown in Columbia broth, then prepared for electroporation by the following steps. The growth was pelleted by centrifugation at 5000 x g for 15 minutes and washed once in 100 ml 272 mM sucrose at 0°C. The cell pellet was resuspended (1:3 packed bacteria:272 mM sucrose) on ice. Competent bacteria (100 µl) were mixed in a 0.1 cm electroporation cuvette (Bio-Rad) with 200 ng plasmid DNA which was either unmethylated or methylated *in vitro* using *Hha*I*.* Immediately after adding DNA, the cells were electroporated (Gene pulser, Bio-Rad) at 18,000 V/cm, 800 ohm, and 25 mFd, with resultant time constants ranging from 11 to 15 msec.

Columbia broth (1 ml, 0°C) was added to the electroporated cells, and the resuspensions were incubated at 25°C for approximately 25 minutes. The cells recovered at 30 °C for 2 hours and were then plated onto Columbia agar plates containing 50 µg/ml kanamycin. Colonies were visible after 24-hour incubation at 30°C. Transformed cells were grown at 30°C overnight in broth containing kanamycin. The cells were then plated onto dextrose starch agar plates with 50 µg/ml kanamycin and grown at 3 °C for 24 hours.

Cells possessing integrated plasmid survived antibiotic selection at the non-permissive temperature for plasmid replication, and these could be identified because integration of replacement plasmid resulted in an acapsular phenotype. Distinguishing between capsular and acapsular *P. multocida* colonies of strains 1059 and 1062 grown on dextrose starch agar plates was easily accomplished. Wild-type capsular colonies of the two strains possess hyaluronic acid, the major capsule component, which renders colonies mucoid in appearance; when viewed under obliquely transmitted light, these colonies exhibit pearl-like iridescence. In contrast, the acapsular single-crossover mutants are non-mucoid and non-iridescent.

Single-crossover mutants were transferred to 5 ml Columbia broth without antibiotic supplementation and incubated at 30 °C overnight to resolve plasmid from the chromosome. Growth was transferred to dextrose-starch agar plates without supplemental antibiotic which were incubated at 38 °C for 12 hours. The initial test to identify double crossover mutants involved replica-plating arrays of cells that appeared acapsular onto dextrose starch agar plates, either with or without antibiotic, and growing the cells at 38°C. Acapsular colonies that failed to grow on the antibiotic plates were subjected to PCR analysis using the *hyaE* forward and reverse primers. The sizes of the PCR products were compared to those of the wild-type parent using agarose gel electrophoresis.

Suspected *P. multocida* 1059 and 1062 h*yaE* deletion mutants were also assayed for the presence of the kanamycin resistance gene. Putative mutants possessing "clean" *hyaE* deletions were devoid of antibiotic resistance sequences. A disc diffusion enzyme assay (Kirby-Bauer test, Bauer *et al., Am. J. Clin. Path. 45,* 493-96, 1966) and an Indian ink staining assay (Collins & Lyne, MICROBIOLOGICAL METHODS, Butterworths, Boston, Mass., 1976, p.110) were used to confirm that the two *hyaE* mutants possessed an acapsular phenotype.

### EXAMPLE 2

### Vaccination of turkey poults with the 1059 ΔhyaE P. multocida strain

Attenuation of the 1059 *ΔhyaE P. multocida* strain was assessed in three week-old broad-breasted white turkey poults. Groups consisting of four poults were injected intramuscularly with ten-fold serial dilutions of exponential growth-phase cultures of wild-type or acapsular mutant cultures suspended in trypticase broth (0.1 ml). The poults were observed for 7 days after challenge.

The LD₅₀ values (*i.e*., the amount of bacteria needed to kill half the poults) were <10³ organisms for poults injected with the wild-type strain and in excess of 10⁷ organisms for poults injected with the *hyaE* mutant.

### EXAMPLE 3

### Vaccination of steer calves with the 1062 ΔhyaE P. multocida strain

Six Holstein steer calves, approximately 500 pounds, were exposed to the 1062 *ΔhyaE P. multocida* acapsular mutant. The strain was administered either by subcutaneous injection in the neck (10⁸ cfu) or by top-dressing feed (10⁹ cfu).

No adverse reaction to either exposure was observed. Mucosal vaccinates became colonized in the palatine tonsils for the remainder of the trial (5 weeks). Intratracheal challenge with the virulent parent strain (10¹⁰ cfu total dose in 15 ml) elicited transient (1-2) day fevers in control calves but little or no pneumonic changes 10 days post-challenge in controls or vaccinates.

## Claims

1. An isolated *Pasteurella multocida* (*P. multocida*) bacterium of serogroup A which comprises a deletion of all or a part of a *hyaE* gene, wherein the deletion attenuates the bacterium.

2. The bacterium of claim 1 which comprises no antibiotic resistance genes.

3. The bacterium of claim 1 which comprises no exogenous DNA.

4. The bacterium of claim 1 which is serotype A:3.

5. The bacterium of claim 1 which is serotype A:1.

6. The bacterium of claim 1 which is serotype A:4.

7. The bacterium of claim 1 wherein amino acids 239-359 of the hyaE protein are deleted.

8. The bacterium of claim 1 which comprises the nucleotide sequence shown in SEQ ID NO:7.

9. The bacterium of claim 1 which comprises the nucleotide sequence shown in SEQ ID NO:8.

10. The bacterium of claim 1 which is live.

11. The bacterium of claim 1 which is lyophilized.

12. The bacterium of claim 1 which is killed.

13. A vaccine for inducing protective immunity against wild-type *P. multocida* comprising:
the bacterium of any one of claims 1 to 12; and
a pharmaceutically acceptable vehicle.

14. The vaccine of claim 13 which is packaged with instructions for administering the vaccine to an ungulate to confer protective immunity against wild-type *P. multocida.*

15. The vaccine of claim 13 which is packaged with instructions for administering the vaccine to a bird to confer protective immunity against wild-type *P. multocida.*

16. A feed suitable for ungulates comprising the bacterium of any one of claims 1 to 12.

17. The feed of claim 16 which is packaged with instructions for administering the feed to ungulates to confer protective immunity against wild-type *P. multocida.*

18. A feed suitable for a bird comprising the bacterium of any one of claims 1 to 12.

19. The feed of claim 18 which is packaged with instructions for administering the feed to a bird to confer protective immunity against wild-type *P. multocida.*

20. Use of the bacterium according to any one of claims 1 to 12 in the manufacture of a medicament for conferring protective immunity against wild-type *P. multocida* to an ungulate or a bird.

21. The use of claim 20 wherein the medicament is for administration to an ungulate.

22. The use of claim 21 wherein the ungulate is a bovine animal.

23. The use of claim 20 wherein the medicament is for administration to a bird.

24. The use of claim 20 wherein the medicament is for administration to a bird and the bird is a poultry bird.

25. The use of claim 20 wherein the medicament is for administration to a bird and the bird is a poultry bird selected from the group consisting of chicken, turkey, ostrich, game hen, squab, guinea fowl, pheasant, quail, duck, goose, and emu.

26. The use of claim 20 wherein the medicament is for administration to a bird and the bird is a chicken.

27. The use of claim 20 wherein the medicament is for administration to a bird and the bird is a turkey.

28. The use of claim 20 wherein the medicament is for administration subcutaneously.

29. The use of claim 20 wherein the medicament is for administration intramuscularly.

30. The use of claim 20 wherein the medicament is for administration orally.

31. The use of claim 20 wherein the medicament is for administration by top-dressing feed.

32. The use of claim 20 wherein the medicament is for administration intranasally.

33. The use of claim 20 wherein the medicament is for administration at a dose between about 10⁴ and about 10⁹ cfu.

34. The use of claim 20 wherein the medicament is for administration at a dose between about 10⁴ and about 10⁶ cfu.

## Patentansprüche

1. Isoliertes *Pasteurelle multocida* (*P*. *multocida*)-Bakterium der Serogruppe A, das eine teilweise oder vollständige Deletion des *hyaE*-Gens umfasst, wobei die Deletion das Bakterium schwächt.

2. Bakterium nach Anspruch 1, das keine Antibiotika-Resistenzgene umfasst.

3. Bakterium nach Anspruch 1, das keine exogene DNA umfasst.

4. Bakterium nach Anspruch 1 vom Serotyp A:3.

5. Bakterium nach Anspruch 1 vom Serotyp A:1.

6. Bakterium nach Anspruch 1 vom Serotyp A:4.

7. Bakterium nach Anspruch 1, wobei die Aminosäuren 239-359 des hyaE-Proteins deletiert sind.

8. Bakterium nach Anspruch 1, das die in SEQ ID NO:7 gezeigte Nukleotidsequenz umfasst.

9. Bakterium nach Anspruch 1, das die in SEQ ID NO:8 gezeigte Nukleotidsequenz umfasst.

10. Bakterium nach Anspruch 1, das lebendig ist

11. Bakterium nach Anspruch 1, das gefriergetrocknet ist.

12. Bakterium nach Anspruch 1, das getötet wurde.

13. Impfstoff zur Erzeugung einer protektiven Immunität gegen Wildtyp-*P*. *multocida,* umfassend
- das Bakterium nach einem der Ansprüche 1 bis 12 und
- ein pharmazeutisch zulässiges Konstituens.

14. Impfstoff nach Anspruch 13, verpackt mit Anweisungen zur Verabreichung des Impfstoffs an ein Huftier, um diesem protektive Immunität gegen Wildtyp-P. *multocida* zu verleihen.

15. Impfstoff nach Anspruch 13, verpackt mit Anweisungen zur Verabreichung des Impfstoffs an einen Vogel, um diesem protektive Immunität gegen Wildtyp-*P. multocida* zu verleihen.

16. Für Huftiere geeignetes, das Bakterium nach einem der Ansprüche 1 bis 12 enthaltendes Futter.

17. Futter nach Anspruch 16, verpackt mit Anweisungen zur Verabreichung des Futters an Huftiere, um diesen protektive Immunität gegen Wildtyp-*P. multocida* zu verleihen.

18. Für Vögel geeignetes, das Bakterium nach einem der Ansprüche 1 bis 12 enthaltendes Futter.

19. Futter nach Anspruch 16, verpackt mit Anweisungen zur Verabreichung des Futters an einen Vogel, um diesem protektive Immunität gegen Wildtyp-*P. multocida* zu verleihen.

20. Verwendung des Bakteriums nach einem der Ansprüche 1 bis 12 bei der Herstellung eines Arzneimittels, das einem Huftier oder Vogel protektive Immunität gegen Wildtyp-*P*. *multocida* verleiht.

21. Verwendung nach Anspruch 20, wobei das Arzneimittel zur Verabreichung an ein Huftier bestimmt ist.

22. Verwendung nach Anspruch 21, wobei das Huftier ein Rind ist.

23. Verwendung nach Anspruch 20, wobei das Arzneimittel zur Verabreichung an einen Vogel bestimmt ist.

24. Verwendung nach Anspruch 20, wobei das Arzneimittel zur Verabreichung an einen Vogel bestimmt und der Vogel ein Geflügeltier ist.

25. Verwendung nach Anspruch 20, wobei das Arzneimittel zur Verabreichung an einen Vogel bestimmt und der Vogel ein Geflügeltier, ausgewählt aus der aus Huhn, Truthahn, Strauß, Wildhuhn, Jungtaube, Perlhuhn, Fasan, Wachtel, Ente, Gans und Emu bestehenden Gruppe, ist.

26. Verwendung nach Anspruch 20, wobei das Arzneimittel zur Verabreichung an einen Vogel bestimmt und der Vogel ein Huhn ist.

27. Verwendung nach Anspruch 20, wobei das Arzneimittel zur Verabreichung an einen Vogel bestimmt und der Vogel ein Truthahn ist.

28. Verwendung nach Anspruch 20, wobei das Arzneimittel zur subkutanen Verabreichung bestimmt ist.

29. Verwendung nach Anspruch 20, wobei das Arzneimittel zur intramuskulären Verabreichung bestimmt ist.

30. Verwendung nach Anspruch 20, wobei das Arzneimittel zur oralen Verabreichung bestimmt ist.

31. Verwendung nach Anspruch 20, wobei das Arzneimittel zur Verabreichung durch Auflegen auf das Futter bestimmt ist.

32. Verwendung nach Anspruch 20, wobei das Arzneimittel zur intranasalen Verabreichung bestimmt ist.

33. Verwendung nach Anspruch 20, wobei das Arzneimittel zur Verabreichung mit einer Dosis von etwa 10⁴ bis etwa 10⁹ koloniebildenden Einheiten (cfu) bestimmt ist.

34. Verwendung nach Anspruch 20, wobei das Arzneimittel zur Verabreichung mit einer Dosis von etwa 10⁴ bis etwa 10⁶ koloniebildenden Einheiten (cfu) bestimmt ist.

## Revendications

1. Bactérie Pasteurella multocida (P. multocida) isolée de sérogroupe A qui comprend une délétion de tout ou partie d'un gène hyaE, dans laquelle la délétion atténue la bactérie.

2. Bactérie selon la revendication 1, qui ne comprend pas de gène de résistance aux antibiotiques.

3. Bactérie selon la revendication 1, qui ne comprend pas d'ADN exogène.

4. Bactérie selon la revendication 1, qui est de sérotype A:3.

5. Bactérie selon la revendication 1, qui est de sérotype A:1.

6. Bactérie selon la revendication 1, qui est de sérotype A:4.

7. Bactérie selon la revendication 1,
**caractérisée en ce que**
les acides aminés 239 à 359 de la protéine hyaE sont supprimés.

8. Bactérie selon la revendication 1, qui comprend la séquence de nucléotides présentée dans SEQ ID NO. 7.

9. Bactérie selon la revendication 1, qui comprend la séquence de nucléotides présentée dans SEQ ID NO. 8.

10. Bactérie selon la revendication 1, vivante.

11. Bactérie selon la revendication 1, lyophilisée.

12. Bactérie selon la revendication 1, tuée.

13. Vaccin destiné à induire une immunité protectrice contre P. multocida de type sauvage comprenant :
la bactérie selon l'une quelconque des revendications 1 à 12 ; et
un véhicule acceptable sur le plan pharmaceutique.

14. Vaccin selon la revendication 13, conditionné accompagné d'instructions pour administrer le vaccin à un ongulé pour conférer une immunité protectrice contre P. multocida de type sauvage.

15. Vaccin selon la revendication 13, conditionné accompagné d'instructions pour administrer le vaccin à un oiseau pour conférer une immunité protectrice contre P. multocida de type sauvage.

16. Aliment adapté aux ongulés comprenant la bactérie selon l'une quelconque des revendications 1 à 12.

17. Aliment selon la revendication 16, conditionné accompagné d'instructions pour administrer l'aliment aux ongulés pour conférer une immunité protectrice contre P. multocida de type sauvage.

18. Aliment adapté à un oiseau comprenant la bactérie selon l'une quelconque des revendications 1 à 12.

19. Aliment selon la revendication 18, conditionné accompagné d'instructions pour administrer l'aliment à un oiseau pour conférer une immunité protectrice contre P. multocida de type sauvage.

20. Utilisation de la bactérie selon l'une quelconque des revendications 1 à 12, dans la fabrication d'un médicament destiné à conférer à un ongulé ou à un oiseau une immunité protectrice contre P. multocida de type sauvage.

21. Utilisation selon la revendication 20,
**caractérisée en ce que**
le médicament est destiné à être administré à un ongulé.

22. Utilisation selon la revendication 21,
**caractérisée en ce que**
l'ongulé est un bovin.

23. Utilisation selon la revendication 20,
**caractérisée en ce que**
le médicament est destiné à être administré à un oiseau.

24. Utilisation selon la revendication 20,
**caractérisée en ce que**
le médicament est destiné à être administré à un oiseau et cet oiseau est une volaille.

25. Utilisation selon la revendication 20,
**caractérisée en ce que**
le médicament est destiné à être administré à un oiseau et cet oiseau est une volaille choisie dans le groupe constitué par les poulets, les dindes, les autruches, les coquelets, les pigeonneaux, les pintades, les faisans, les cailles, les canards, les oies et les émeus.

26. Utilisation selon la revendication 20,
**caractérisée en ce que**
le médicament est destiné à être administré à un oiseau et cet oiseau est un poulet.

27. Utilisation selon la revendication 20,
**caractérisée en ce que**
le médicament est destiné à être administré à un oiseau et cet oiseau est une dinde.

28. Utilisation selon la revendication 20,
**caractérisée en ce que**
le médicament est destiné à une administration sous-cutanée.

29. Utilisation selon la revendication 20,
**caractérisée en ce que**
le médicament est destiné à une administration intramusculaire.

30. Utilisation selon la revendication 20,
**caractérisée en ce que**
le médicament est destiné à une administration orale.

31. Utilisation selon la revendication 20,
**caractérisée en ce que**
le médicament est destiné à une administration par saupoudrage sur des aliments.

32. Utilisation selon la revendication 20,
**caractérisée en ce que**
le médicament est destiné à une administration intranasale.

33. Utilisation selon la revendication 20,
**caractérisée en ce que**
le médicament est destiné à être administré à une dose comprise entre environ 10⁴ et environ 10⁹ UFC.

34. Utilisation selon la revendication 20,
**caractérisée en ce que**
le médicament est destiné à être administré à une dose comprise entre environ 10⁴ et environ 10⁶ UFC.


### SEQUENCE LISTING

<110> Biotechnology Research Development Corporation
   The United States of America, as Represented by the Department of Agriculture
<120> Acapsular *P. multocida hyaE* Deletion Mutants
<151> 2003-07-02
<160> 9
<170> FastSEQ for Windows Version 4.0
<210> 1
   <211> 23
   <212> DNA
   <213> P. multocida
<400> 1
   atgaaaaagg ttaatatcat tgg 23
<210> 2
   <211> 24 ,
   <212> DNA
   <213> P. multocida
<400> 2
   ttaaccttgc ttgaatcgtt tacc 24
<210> 3
   <211> 30
   <212> DNA
   <213> P. multocida
<400> 3
   aaagatatct tggtttactt caataatttc 30
<210> 4
   <211> 33
   <212> DNA
   <213> P. multocida
<400> 4
   aaagatatca ctgcatctgt tcaatcaacg agc 33
<210> 5
   <211> 1870
   <212> DNA
   <213> P. multocida
<400> 5
   tatgaaaaag gttattatca ttggacataa acagtctaac tatcaagatg ttgaaaaggt     60
   ttttcaatgt tatgggatga atcccccgct tccatcaaaa cgtgaaaaaa tgtcccccat    120
   cgaaattggc catgtgctta ataaagtatt accaagtctt gagcacacac ctaaaaatgt    180
   atctttactt tctaataaga aaagcaaaat aaaaaaaggg aattcagcca aaaataaatc    240
   tcataagcac gctaaaacga acacaataca aacgacttcg agcatctggg ataacttatc    300
   tctcgatttg atgctcgcga atatcgagca aaatttttgg ggatggtctg atcctaatgc    360
   aattcaaata ttagattatt gggctaacct tgacccaaac attcatttcg tctttgttta    420
   tgataagcca gaaaatttat tccaatatca tagcttagaa gaggctctca aattagataa    480
   acacaccgta caagaaaaat ttgaagagtg gcaaacctac aatgaaaaaa tcctaactta    540
   ctttaataaa tataaagatc gtagtgtatt actgaataca caacaactcc aaaatactaa    600
   aaaaacatca ctgtctgaaa tttataaaca tatttctgca cctgatgcat tagtcaaaaa    660
   actgaatgaa ccttctctaa ataaagagat ggaaattatt gaagtaaacc aagatttatc    720
   tcaccaagaa gaatgtccac tgtctaactt tattgttagc caaattataa aaaattctcc    780
   tactgttacg caggtatatg aagaattaca gtcgcatgct gatctgcctt atatttcaga    840
   acaaaaatta gtaaatgatg ccgattttgc tctccttgca tggaaagata tgattcaaaa    900
   acaagtcgat gcaaatcaat atcaacatga aaaagaatta gaacttagca caataaaaga    960
   acgtcaatta gaggtcacag agaaatatca attgacggaa caaaaactgt cagaaacaca   1020
   aaaagaaatc gaacaaatta aagatgaaaa tagaaaagta aaatctgaaa aagcaaaact   1080
   cactgcatct gttcaatcaa cgagcaaaat actttctgag aaagaaaaag agatttcttg   1140
   cataaaaagt gaaaatacaa agattaaaga agaaaaaatt aaaattgatg aagcatacca   1200
   cttaaccaag aaaaccttgt cggataaaga aaaagccctc aaaacgcatc aagatgaaat   1260
   tgaagcgctc aagataattt ttaatgaaaa tatttccgta caagaagata tgcaagaaaa   1320
   atttcaggaa accaataaaa gaaaacaaga acttgaacaa gagctaaaag ccatatcgga   1380
   taagaaagca ttattagaaa cagaaaacag ccaaaaaacc caagtatctg agtctttaga   1440
   aaatgaaaat aaagtgttat tagctcaact ccaactcatt caagaagaat tagaaaaact   1500
   ttatattgac aatcaagtat taaaagctaa accacgcctt tacggtgcag ctgatcgcat   1560
   aaaaaaccaa ttaacttatc gactaggtta caaaatacaa agacatggaa gaagtctatt   1620
   tggtctcatt tttcttcctt tcatcttatt tttcacctat ctgggcttta aaagagagat   1680
   gaaaaagtac gaatggaata ccctcccacc aattcacgag tatgaagacg cgcataaagc   1740
   aaaccgcatt aaaagccatt tatcttataa attgggtgtc atctttttac aagaaatcaa   1800
   aaatccgttt aagtggctta ctctccctta caaactgatt aaagaaggta aacgattcaa   1860
   gcaaggttaa                                                          1870
<210> 6
   <211> 1870
   <212> DNA
   <213> P. multocida
<400> 6
   tatgaaaaag gttattatca ttggacataa acagtctaac tatcaagatg ttgaaaaggt     60
   ttttcaatgt tatgggatga atcccccgct tccatcaaaa cgtgaaaaaa tgtcccccat    120
   cgaaattggc catgtgctta ataaagtatt accaagcctt gagcacacac ctaaaaatgt    180
   atctttactt tctaataaga aaagcaaaat aaaaaaaggg aattcagcca aaaataaatc    240
   tcataagcac gctaaaacga acacaataca aacgacttcg agcatctggg ataacttatc    300
   tctcgatttg atgctcgcga atatcgagcg aaatttttgg ggatggtctg atcctaatgc    360
   aattcaaata ttagattatt gggctaacct cgacccaaac attcatttcg tctttgttta    420
   tgataagcca gaaaatttat tccaatatca tagcttagaa gaggctctca aattagataa    480
   acacaccgta caagaaaaat ttgaagagtg gcaaacctac aatgaaaaaa tcctaactta    540
   ctttaataaa tataaagatc gtagtgtatt actgaataca caacaactcc aaaatactaa    600
   aaaaacatca ctgtctgaaa tttataaaca tatttctgca cctgatgcat tagtcaaaaa    660
   actgaatgaa ccttctctaa ataaagagat ggaaattatt gaagtaaacc aagatttatc    720
   tcaccaagaa gaatgtccac tgtctaactt tattgttagc caaattataa aaaattctcc    780
   tactgttacg caggtatatg aagaattaca gtcgcatgct gatctgcctt atatttcaga    840
   acaaaaatta gtaaatgatg ccgattttgc tctccttgca tggaaagata tgattcaaaa    900
   acaagtcgat gcaaatcaat atcaacatga aaaagaatta gaacttagca caataaaaga    960
   acgtcaatta gaggtcacag agaaatatca attgacggaa caaaaactgt cagaaacaca   1020
   aaaagaaatc gaacaaatta aagatgaaaa tagaaaagta aaatctgaaa aagcaaaact   1080
   cactgcatct gttcaatcaa cgagcaaaat actttctgag aaagaaaaag agatttcttg   1140
   cataaaaagt gaaaatacaa agattaaaga agaaaaaatt aaaattgatg aagcatacca   1200
   cttaaccaag aaaaccttgt cggataaaga aaaagccctc aaaacgcatc aagatgaaat   1260
   tgaagcgctc aagataattt ttaatgaaaa tatttccgta caagaagata tgcaagaaaa   1320
   atttcaggaa accaataaaa gaaaacaaga acttgaacaa gagctaaaag ccatatcgga   1380
   taagaaagca ttattagaaa cagaaaacag ccaaaaaacc caagtatctg agtctttaga   1440
   aaatgaaaat aaagtgttat tagctcaact ccaactcatt caagaagaat tagaaaaact   1500
   ttatattgac aatcaagtat taaaagctaa accacgcctt tacggtgcag ctgatcgcat   1560
   aaaaaaccaa ttaacttatc gactaggtta caaaatacaa agacatggaa gaagtctatt   1620
   tggtctcatt tttcttcccc ttatcttatt ttgcacttat ttgcgtttta aaaaagagat   1680
   gaaaaagtac gaatggaata ccctcccacc aattcacgag tatgaagacg cgcataaagc   1740
   aaaccgcatt aaaagccatt tatcttataa attgggtgtc atctttttac aagaaatcaa   1800
   aaatccgttt aagtggctta ctctccctta caaactgatt aaagaaggta aacgattcaa   1860
   gcaaggttaa                                                          1870
<210> 7
   <211> 1507
   <212> DNA
   <213> P. multocida
<400> 7
   tatgaaaaag gttattatca ttggacataa acagtctaac tatcaagatg ttgaaaaggt     60
   ttttcaatgt tatgggatga atcccccgct tccatcaaaa cgtgaaaaaa tgtcccccat    120
   cgaaattggc catgtgctta ataaagtatt accaagtctt gagcacacac ctaaaaatgt    180
   atctttactt tctaataaga aaagcaaaat aaaaaaaggg aattcagcca aaaataaatc    240
   tcataagcac gctaaaacga acacaataca aacgacttcg agcatctggg ataacttatc    300
   tctcgatttg atgctcgcga atatcgagca aaatttttgg ggatggtctg atcctaatgc    360
   aattcaaata ttagattatt gggctaacct tgacccaaac attcatttcg tctttgttta    420
   tgataagcca gaaaatttat tccaatatca tagcttagaa gaggctctca aattagataa    480
   acacaccgta caagaaaaat ttgaagagtg gcaaacctac aatgaaaaaa tcctaactta    540
   ctttaataaa tataaagatc gtagtgtatt actgaataca caacaactcc aaaatactaa    600
   aaaaacatca ctgtctgaaa tttataaaca tatttctgca cctgatgcat tagtcaaaaa    660
   actgaatgaa ccttctctaa ataaagagat ggaaattatt gaagtaaacc aagatatcac    720
   tgcatctgtt caatcaacga gcaaaatact ttctgagaaa gaaaaagaga tttcttgcat    780
   aaaaagtgaa aatacaaaga ttaaagaaga aaaaattaaa attgatgaag cataccactt    840
   aaccaagaaa accttgtcgg ataaagaaaa agccctcaaa acgcatcaag atgaaattga    900
   agcgctcaag ataattttta atgaaaatat ttccgtacaa gaagatatgc aagaaaaatt    960
   tcaggaaacc aataaaagaa aacaagaact tgaacaagag ctaaaagcca tatcggataa   1020
   gaaagcatta ttagaaacag aaaacagcca aaaaacccaa gtatctgagt ctttagaaaa   1080
   tgaaaataaa gtgttattag ctcaactcca actcattcaa gaagaattag aaaaacttta   1140
   tattgacaat caagtattaa aagctaaacc acgcctttac ggtgcagctg atcgcataaa   1200
   aaaccaatta acttatcgac taggttacaa aatacaaaga catggaagaa gtctatttgg   1260
   tctcattttt cttcctttca tcttattttt cacctatctg ggctttaaaa gagagatgaa   1320
   aaagtacgaa tggaataccc tcccaccaat tcacgagtat gaagacgcgc ataaagcaaa   1380
   ccgcattaaa agccatttat cttataaatt gggtgtcatc tttttacaag aaatcaaaaa   1440
   tccgtttaag tggcttactc tcccttacaa actgattaaa gaaggtaaac gattcaagca    1500
   aggttaa                                                              1507
<210> 8
   <211> 1531
   <212> DNA
   <213> P. multocida
<400> 8
   tatgaaaaag gttattatca ttggacataa acagtctaac tatcaagatg ttgaaaaggt      60
   ttttcaatgt tatgggatga atcccccgct tccatcaaaa cgtgaaaaaa tgtcccccat     120
   cgaaattggc catgtgctta ataaagtatt accaagcctt gagcacacac ctaaaaatgt     180
   atctttactt tctaataaga aaagcaaaat aaaaaaaggg aattcagcca aaaataaatc     240
   tcataagcac gctaaaacga acacaataca aacgacttcg agcatctggg ataacttatc     300
   tctcgatttg atgctcgcga atatcgagcg aaatttttgg ggatggtctg atcctaatgc     360
   aattcaaata ttagattatt gggctaacct cgacccaaac attcatttcg tctttgttta     420
   tgataagcca gaaaatttat tccaatatca tagcttagaa gaggctctca aattagataa     480
   acacaccgta caagaaaaat ttgaagagtg gcaaacctac aatgaaaaaa tcctaactta     540
   ctttaataaa tataaagatc gtagtgtatt actgaataca caacaactcc aaaatactaa     600
   aaaaacatca ctgtctgaaa tttataaaca tatttctgca cctgatgcat tagtcaaaaa     660
   actgaatgaa ccttctctaa ataaagagat ggaaattatt gaagtaaacc aagatccccg     720
   gggccccggg gccccgggga tcactgcatc tgttcaatca acgagcaaaa tactttctga     780
   gaaagaaaaa gagatttctt gcataaaaag tgaaaataca aagattaaag aagaaaaaat     840
   taaaattgat gaagcatacc acttaaccaa gaaaaccttg tcggataaag aaaaagccct     900
   caaaacgcat caagatgaaa ttgaagcgct caagataatt tttaatgaaa atatttccgt     960
   acaagaagat atgcaagaaa aatttcagga aaccaataaa agaaaacaag aacttgaaca    1020
   agagctaaaa gccatatcgg ataagaaagc attattagaa acagaaaaca gccaaaaaac    1080
   ccaagtatct gagtctttag aaaatgaaaa taaagtgtta ttagctcaac tccaactcat    1140
   tcaagaagaa ttagaaaaac tttatattga caatcaagta ttaaaagcta aaccacgcct    1200
   ttacggtgca gctgatcgca taaaaaacca attaacttat cgactaggtt acaaaataca    1260
   aagacatgga agaagtctat ttggtctcat ttttcttccc cttatcttat tttgcactta    1320
   tttgcgtttt aaaaaagaga tgaaaaagta cgaatggaat accctcccac caattcacga    1380
   gtatgaagac gcgcataaag caaaccgcat taaaagccat ttatcttata aattgggtgt    1440
   catcttttta caagaaatca aaaatccgtt taagtggctt actctccctt acaaactgat    1500
   taaagaaggt aaacgattca agcaaggtta a                                   1531
<210> 9
   <211> 8
   <212> PRT
   <213> Artificial Sequence
<220>
   <223> inserted amino acids
<400> 9
   Pro Arg Gly Pro Gly Ala Pro Gly
   1               5